(19) Europäisches Patentamt
European Patent Office
Office européen des brevets

(11) Publication number: 0 054 428 A1

(12) # EUROPEAN PATENT APPLICATION

(21) Application number: 81305868.2

(51) Int. Cl.³: C 07 D 207/32

(22) Date of filing: 14.12.81

(30) Priority: 15.12.80 US 216665

(43) Date of publication of application: 23.06.82 Bulletin 82/25

(84) Designated Contracting States: BE CH DE FR GB IT LI NL SE

(71) Applicant: ETHYL CORPORATION
330 South Fourth Street
Richmond Virginia(US)

(72) Inventor: Nelson, Gunner Elwood
12646 Lockhaven Avenue
Baton Rouge Louisiana(US)

(74) Representative: Baillie, Iain Cameron et al,
c/o Ladas & Parry Isartorplatz 5
D-8000 München 2(DE)

(54) **Preparation of pyrrole esters.**

(57) A process for preparing substituted pyrrole-2-acetic acid, salt or ester intermediates by treating a 1-$R_4$-4-$R_5$-3-$R_8$-pyrrole-2-acetic acid, salt or ester with base at elevated temperatures. The substituted pyrrole-2-acetic acid, salt or ester intermediate is useful for preparation of anti-inflammatory and analgesic compounds.

EP 0 054 428 A1

Croydon Printing Company Ltd.

PREPARATION OF PYRROLE ESTERS

This invention relates to a process for producing 5-aroyl-pyrroles and, more particularly, to 1,4-dialkyl-5-aroyl-pyrrole alkanoic acids and the corresponding salts, esters, nitriles, amides and substituted amides thereof.

Such 5-aroyl-pyrroles are known as effective anti-inflammatory compounds having useful pharmacological properties and which are suitable for incorporation into conventional pharmaceutical forms for administration. The anti-inflammatory activity has been demonstrated in the standard kaolin-induced rat paw edema and cotton pellet granuloma tests at doses generally ranging from 5-100 mg/kg body weight. The compounds produced by the process of the present invention can be more particularly illustrated by reference to prior art patents in this particular art.

Specifically, Carson U. S. 3,752,826 teaches a number of compounds in the class of 5-aroyl-pyrrole alkanoic acids and the corresponding acid derivatives thereof which are useful as anti-inflammatory agents and as synthetic intermediates. Particularly, Carson teaches the compounds per se and methods for their preparation.

U. S. 3,865,840 and 3,952,012, which are divisions of Carson's U. S. 3,752,826, are particularly directed to the process of making loweralkyl 1,4-diloweralkyl-3-lower alkoxy carbonyl-pyrrole-2-acetate compounds and a process of making a loweralkyl 5-aroyl-1-$R_4$-4-$R_5$-pyrrole-2-acetate in which $R_4$ and $R_5$ represent lower alkyl compounds. The Carson '840 patent teaches a process of reacting, at just below 60°C., a

mixture of a loweralkyl amine of the formula $R_4-NH_2$ and a dilower alkyl acetone dicarboxylate of the formula:

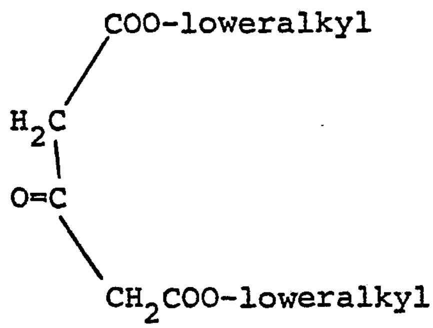

with a chloromethyl loweralkyl ketone of the formula: $Cl-CH_2-CO-R_5$, wherein the foregoing formulas said $R_4$ and said $R_5$ represent loweralkyl. The resulting pyrrole diester is then employed according to the teaching of the Carson '012 patent to produce the loweralkyl 5-aroyl-1-$R_4$-4-$R_5$-pyrrole-2-acetate by the steps of (1) hydrolyzing the diester under alkaline conditions to give a diacid, (2) partially re-esterifying with an acidic solution of a lower alkanol to give a loweralkyl 1-$R_4$-4-$R_5$-3-carboxy-pyrrole-2-acetate, (3) decarboxylating the 3-carboxy group to form a pyrrole ester by heating the partially re-esterified pyrrole to carbon dioxide elimination temperatures and (4) acylating the lower 1-$R_4$-4-$R_5$-pyrrole-2-acetate with an aroyl chloride in the presence of a Lewis acid in an organic solvent suitable for Friedel-Crafts acylation reactions to obtain the desired loweralkyl 5-aroyl-1-$R_4$-4-$R_5$-pyrrole-2-acetate.

The process for preparation of such pyrroles having an alkyl substituent in the 4-position is extremely difficult after the pyrrole ring is formed. This is especially true when the ring contains other substituents. Accordingly, the '012 patent teaches that it is necessary for the formation of the 4-loweralkyl-substituted 5-aroyl-pyrroles to proceed through the laborious sequence of ring formation producing the diester, hydrolysis of the diester to the diacid, partial re-esterification to the 2-acetate, decarboxylation of the 3-carboxy group and then hydrolysis to the acid. In an alternate procedure the acylation to the 5-aroyl-pyrrole diester can take place after ring formation of the pyrrole

diester, followed by the above-described sequence of hydrolysis, partial re-esterification, decarboxylation and hydrolysis. More specific disclosure of the starting materials and prior art process sequences can be obtained by reference to U. S. 3,752,826, U. S. 3,865,840 and U. S. 3,952,012.

The present invention provides a process for the preparation of a compound of the formula:

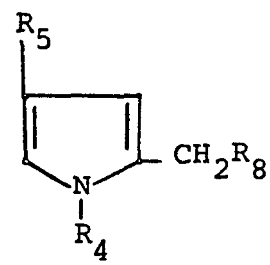

which comprises converting a substituted pyrrole compound of formula:

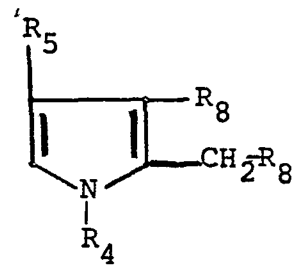

with base at elevated temperatures wherein the foregoing formulas said $R_4$ and said $R_5$ each are independently selected from loweralkyl groups, and each said $R_8$ is independently selected from COOH, $COOR_4$, and COOM in which said M is an alkali metal or alkaline earth metal.

The present process preferably employs a pyrrole diester from the ring-formation step in a process for producing 5-aroyl-pyrrole alkanoic acids, or any suitable pyrrole compound having a carboxylic acid, carboxylate ester or carboxylate salt group substituted at the 2- or 3-position, such as a 3-alkoxy-carbonyl-pyrrole-2-acetate, a 3-carboxypyrrole-2-acetate, a 3-carboxy-pyrrole-2-acetic acid, a 3-carboxypyrrole-2-acetic acid alkali metal salt, a 3-alkoxycarbonylpyrrole-2-acetic acid alkali metal salt, a 3-carboxypyrrole-2-acetic acid dialkali metal salt and the like.

Preferably, the present process employs a lower alkyl pyrrole diester. More preferably, the pyyrole starting diester is a precursor for valuable pharmaceutical products, as indicated in the prior art hereinabove cited and incorporated herein. Thus, the starting pyrrole diester and, as well, the other pyrrole compounds include a pyrrole ring having the nitrogen atom substituted with a lower alkyl group which can be methyl, ethyl, propyl or butyl. Similarly, the 4-position of the pyrrole ring is substituted with a lower alkyl group. The pyrrole ring is substituted in the 2-position with an alkylene chain bearing an ester group. The alkylene chain can be one or more carbon atoms. Thus, the ester group in the 2-position can be an acetate, a propionate, or butyrate group, although the acetate group is preferred. The other ester group is attached at the 3-position and the carbonyl carbon atom is directly attached to the pyrrole ring. Both ester groups can have, as the alcohol portion thereof, a lower alkyl group such as methyl, ethyl, propyl, or butyl. The 5-position can be unsubstituted or substituted with an aroyl group of the character recited in the prior art patents. Specifically, if the pyrrole ring is substituted in the 5-position, the aryl group which is connected to the pyrrole ring by a carbonyl carbon atom is a member selected from the group consisting of phenyl, thienyl, 5-methyl-thienyl, monosubstituted phenyl and polysubstituted phenyl, each substituent of the substituted phenyl groups being a member selected from the group consisting of halo, loweralkyl, trifluoromethyl, loweralkoxy, nitro, amino, cyano and methylthio. In order not to lose valuable and expensive reagents, it is generally considered desirable to have the pyrrole diester, or other pyrrole reactant compound, unsubstituted in the 5-position until it is converted to the monoester. The pyrrole diester starting material may be employed in any convenient form. For example, it may be in its pure form as a white solid or it may be in a solution or crude mixture with a liquid carried over from the process in which it is produced. A more preferred pyrrole compound useful in the present process is the pyrrole diacid, such as for example, 1,4-dimethyl-3-carboxypyrrole-2-acetic acid.

The pyrrole diester or other pyrrole reactant compound is reacted with a mixture containing a base. The base can be any strong base. Typical of a useful base is the alkali metal hydroxides and alkaline earth metal hydroxides. Specifically, sodium hydroxide, potassium hydroxide, rubidium hydroxide, magnesium hydroxide, calcium hydroxide, strontium hydroxide and the like are illustrative examples of alkali metal and alkaline earth metal hydroxides. Preferably, an aqueous base can be employed, of which, aqueous caustic and aqueous potassium hydroxide are especially preferred.

The amount of base employed should be sufficient to effect the decarboxylation of the pyrrole compound. Specifically, it has been found that an amount of base ranging from a molar equivalent to about three times the molar equivalent amount of pyrrole compound can be used. Preferably, an amount of base about twice the molar equivalent of the amount of pyrrole compound has been found to react favorably in the present process. Thus, from 1 to about 3 moles of base per mole of pyrrole compound can be used in the present process.

The process of the present invention can be carried out at elevated temperatures. However, the temperature should not be so high as to degrade the products. In general, as the temperature increases, an increase in the rate of the reaction is observed. Temperatures from 150°C. up to 180°C. have been found to be useful. However, decarboxylation temperatures of 210-230°C., which were employed in prior art processes, have been found to adversely affect the product and substantially reduce the yields. Preferably, the temperature should be maintained at between 160 and 180°C. depending on the length of time the reaction is to be run.

The pyrrole reactant compound, preferably the pyrrole diester, can be placed in the reaction vessel and the base added thereto. The reaction mixture is then heated with agitation for a period of time to give the desired pyrrole

product.

The product can be recovered as a salt by extraction into an organic phase. Recovery of the product should be carried out so that handling losses are minimized and degradation by-products are not produced. In one recovery method, the pH is adjusted with acid to about pH 7.5, and the solution evaporated to dryness. The residual salts were slurried in a suitable solvent, such as DMF, and the slurry was treated with ethyl bromide to convert the salt to the corresponding ester. Extraction with water and organic solvent, drying and evaporation of the solvent affords the decarboxylated pyrrole monoester. The product pyrrole monoester need not be recovered by stripping the solvent from the product as the product can be carried on for further processing according to the procedures described in the prior art.

Having described the process in general, it is believed that the following examples will illustrate further specific embodiments of the process of the present invention.

EXAMPLE 1

A mixture of 5 g. (0.0254 mole) of 1,4-dimethyl-3-carboxypyrrole-2-acetic acid and 2.02 g. (0.0508 mole) of sodium hydroxide was dissolved in 20 grams of 10 wt. percent caustic solution. The mixture was sealed in a 50 ml. stainless steel pressure vessel and heated for 5 hours at 175°C. After cooling, the resultant solution was treated with concentrated hydrochloric acid until a pH of about 7.5 was reached and the neutralized solution was evaporated to dryness. Vigorous off-gassing was observed during pH adjustment. The evaporated residue was slurried in 25 ml of DMF and treated with 150 mmoles of ethyl bromide at room temperature overnight. The resultant mixture was then diluted with 300 ml of ice water, extracted with 150 ml of chloroform, the chloroform extract was dried over magnesium sulfate and evaporated to yield 26.3 grams of solution which was analyzed by gas chromatograph to contain 5.86 percent ethyl 1,4-dimethylpyrrole-2-acetate for a yield of 33.5

percent.

EXAMPLE 2

A mixture of 5 g. (0.0254 mole) of 1,4-dimethyl-3-carboxypyrrole-2-acetic acid and 2.84 g. (0.0508 mole) of potassium hydroxide in 20 grams of 10 wt. percent potassium hydroxide was sealed in a 50 ml stainless steel pressure vessel and heated for 18 hours at 175°C. in a procedure similar to Example 1. On cooling, the resultant mixture was treated with concentrated hydrochloric acid until the pH was adjusted to about pH 8. The aqueous solution of salts was evaporated to dryness, then reslurried in 25 ml of DMF and treated with 100 mmoles of ethyl bromide with agitation overnight at room temperature. The resultant mixture was filtered and washed with methylene chloride. The solvent was evaporated and 9.3 grams of liquid were recovered. Gas chromatographic analysis indicated 9.3 percent of ethyl 1,4-dimethyl-pyrrole-2-acetate for a 62.7% yield.

Although the examples have been given illustrating the invention with sodium hydroxide and potassium hydroxide as the base, one skilled in the art can plainly see that other reagents can be employed. Similarly, other pyrrole compounds can be used and the optimum conditions can be determined following the procedures outlined hereinabove.

1. A process for preparation of a compound of the formula:

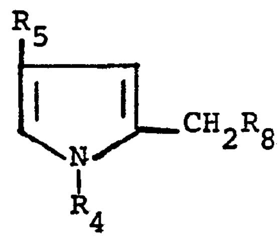

which comprises converting a substituted pyrrole compound of the formula

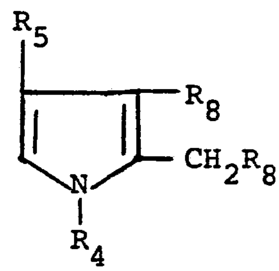

with base at elevated temperatures wherein the foregoing formulas $R_4$ and $R_5$ each are independently selected from lower alkyl and each $R_8$ is independently selected from COOH, $COOR_4$ and COOM in which said M is an alkali metal or alkaline earth metal.

2. The process of claim 1, in which said base is selected from sodium hydroxide and potassium hydroxide.

3. The process of claim 1, in which said temperature ranges from 150° to 190°C.

4. The process of claim 1, in which said base is sodium hydroxide.

5. The process of claim 1, in which said base is potassium hydroxide.

6. The process of claim 1, in which said temperature ranges from 170° to 180°C.

7. The process of claim 1, for preparation of a compound of the formula

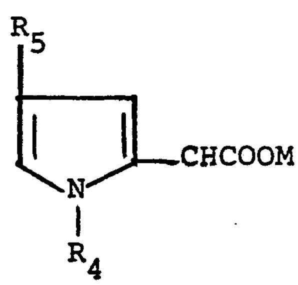

CASE 4853

which comprises contacting a substituted pyrrole compound of the formula

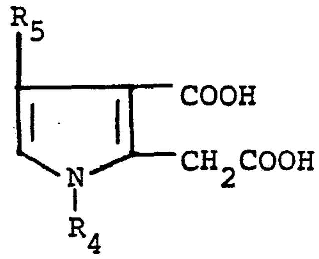

with base at elevated temperatures.

8. The process of claim 7, further characterized in that said base is sodium hydroxide.

9. The process of claim 7, further characterized in that said base is potassium hydroxide.

10. The process of claim 7, further characterized in that said base is sodium hydroxide and said temperature ranges from 150° to 190°C.

0054428

European Patent Office

# EUROPEAN SEARCH REPORT

Application number
EP 81 30 5868

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| X | US - A - 4 070 368 (CARSON)<br>* Column 9,10, formules, XXXII and XXXIII *<br>-- | 1-10 | C 07 D 207/32 |
| X | JOURNAL OF MEDICINAL CHEMISTRY, vol. 16, nr. 1, January 1973<br>J.R. CARSON et al. "5-Benzoyl-1-methylpyrrole-2-acetic Acids as Antiinflammatory Agents.2. The 4-Methyl Compounds", pages 172-175<br>* Page 173, formules VIII and IX *<br>---- | 1-10 | |
| | | | TECHNICAL FIELDS SEARCHED (Int.Cl. 3) |
| | | | C 07 D 207/00 |

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

✓ The present search report has been drawn up for all claims

| Place of search | Date of completion of the search | Examiner |
|---|---|---|
| The Hague | 11-02-1982 | MAISONNEUVE |

EPO Form 1503.1 06.78